# EUROPEAN PATENT SPECIFICATION

(11) **EP 4 382 060 B1**
(45) Date of publication and mention of the grant of the patent: **04.02.2026**
(21) Application number: 23214226.5
(22) Date of filing: 05.12.2023
(51) Int. Cl.: A61B 18/14

(54) **ELECTRODES FOR BASKET CATHETERS**
ELEKTRODEN FÜR KORBKATHETER
ELECTRODES POUR CATHETERS A PANIER

(30) Priority: 06.12.2022 US 202263386278 P; 02.11.2023 US 202318500375
(43) Date of publication of application: 12.06.2024
(73) Proprietor: Biosense Webster (Israel) Ltd., 2066717 Yokneam (IL)
(72) Inventor: JENKINS, Nathaniel, Irvine, 92618 (US); KEYES, Joseph Thomas, Irvine, 92618 (US); HERRERA, Kevin Justin, Irvine, 92618 (US)
(74) Representative: Carpmaels & Ransford LLP

(56) References cited:
- EP-A1- 4 079 244
- US-A- 5 555 618
- US-A1- 2009 149 848
- US-A1- 2015 366 508
- US-A1- 2022 054 198

## Description

### FIELD

The present invention relates generally to medical devices, and in particular medical probes with electrodes, and further relates to, but not exclusively, medical probes suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1.

EP4079244A1 discloses a medical apparatus, which includes a probe, including an insertion tube configured for insertion into a body cavity of a patient and a basket assembly having a proximal end that is connected distally to the insertion tube and including a plurality of resilient spines, which are configured to bow radially outward around a longitudinal axis of the basket assembly and are conjoined at a distal end of the basket assembly.

Regions of cardiac tissue can be mapped by a catheter to identify the abnormal electrical signals. The same or different medical probe can be used to perform ablation. Some example probes include a number of spines with electrodes positioned thereon. The electrodes are generally attached to the spines and secured in place by soldering, welding, or using an adhesive. Due to the small size of the spines and the electrodes, however, soldering, welding, or adhering the electrodes to the spines can be a difficult task, increasing the manufacturing time and cost and the chances that the electrode fails due to an improper bond, misalignment, or strain on the spine. What is needed, therefore, are systems and methods of attaching an electrode to a spine of a basket assembly without the need for soldering, welding, or using adhesive.

### SUMMARY

The invention provides an electrode for a medical probe according to claim 1 and a medical probe according to claim 12. Embodiments are provided by the dependent claims.

### BRIEF DESCRIPTION OF THE DRAWINGS

FIG. 1 is a schematic pictorial illustration of a medical system including a medical probe with a distal end that includes a medical probe with electrodes, in accordance with an embodiment of the present invention;
FIG. 2A is a schematic pictorial illustration showing a perspective view of a medical probe with electrodes in an expanded form, in accordance with an embodiment of the present invention;
FIG. 2B is a schematic pictorial illustration showing a side view of a medical probe in a collapsed form, in accordance with the disclosed technology;
FIG. 3A is a schematic pictorial illustration showing a top view of a spine and an electrode attached thereto, in accordance with the disclosed technology;
FIG. 3B is a schematic pictorial illustration showing a side view of a spine and an electrode attached thereto, in accordance with an embodiment of the present invention;
FIG. 3C is a schematic pictorial illustration showing a front view of an electrode body with legs, in accordance with the disclosed technology;
FIG. 3D is a schematic pictorial illustration showing a front view of an electrode body with legs crimped onto a spine of a medical probe, in accordance with the disclosed technology;
FIG. 4A is a schematic pictorial illustration showing a top view of a spine and an electrode body with an undulating outer surface, flexible material coating, and windows, in accordance with the disclosed technology;
FIG. 4B is a schematic pictorial illustration showing a side view of a spine and an electrode body with an undulating outer surface, flexible material coating, and windows, in accordance with the disclosed technology;
FIG. 4C is a schematic pictorial illustration showing a front view of an electrode body with legs, in accordance with the disclosed technology;
FIG. 4D is a schematic pictorial illustration showing a front view of an electrode body with legs crimped onto a spine of a medical probe, in accordance with the disclosed technology;
FIG. 5A is a schematic pictorial illustration showing a top view of a spine and an electrode body including a spiral wound wire, in accordance with the disclosed technology;
FIG. 5B is a schematic pictorial illustration showing a side view of a spine and an electrode body including a spiral wound wire, in accordance with the disclosed technology;
FIG. 5C is a schematic pictorial illustration showing a front view of a spine and an electrode body including a spiral wound wire, in accordance with the disclosed technology;
FIG. 6A is a schematic pictorial illustration showing a top view of a spine and an electrode having an electrode body that tapers from a proximal end to a distal end, in accordance with the disclosed technology;
FIG. 6B is a schematic pictorial illustration showing a side view of a spine and an electrode having an electrode body that tapers from a proximal end to a distal end, in accordance with the disclosed technology;
FIG. 6C is a schematic pictorial illustration showing a front view of an electrode body that tapers from a proximal end to a distal end and includes legs in accordance with the disclosed technology;
FIG. 6D is a schematic pictorial illustration showing a front view of electrode body that tapers from a proximal end to a distal end and includes legs crimped onto a spine of a medical probe, in accordance with the disclosed technology; and
FIG. 7 is a flowchart illustrating a method of attaching an electrode to a spine, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, method or uses and devices for IRE ablation of cardiac tissue to treat cardiac arrhythmias. Whilst no claim is directed to these methods per se, the systems are capable of being used and are intended to be used in such methods. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and unclaimed methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1.

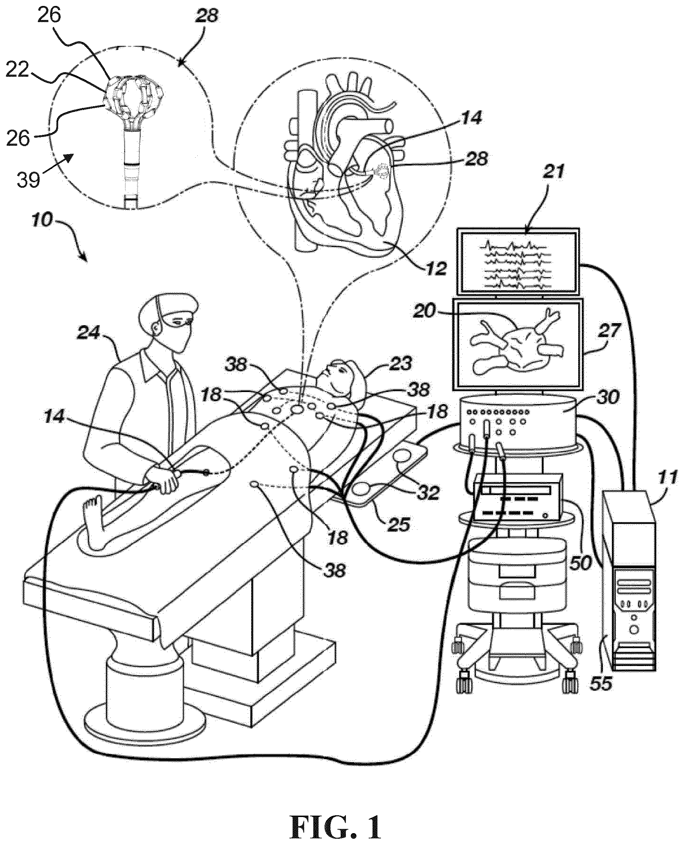

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip of catheter 14 (i.e., a basket catheter 28 in this case) into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over a plurality of spines 22 at basket catheter 28 and configured to sense the IEGM signals. Catheter 14 may additionally include a position sensor 29 embedded in or near basket catheter 28 for tracking position and orientation of basket catheter 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of basket catheter 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6 892,091.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

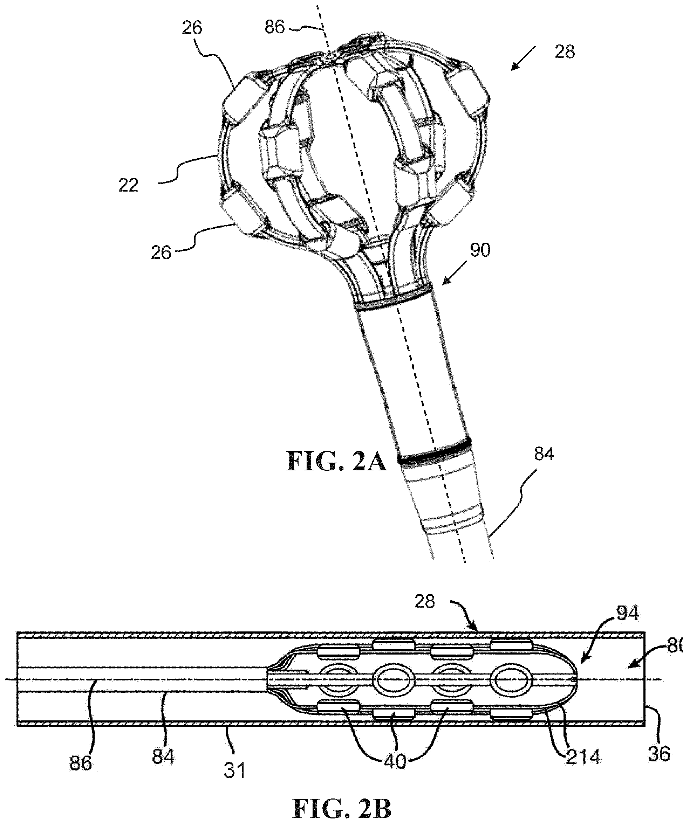

FIG. 2A is a schematic pictorial illustration showing a perspective view of a medical probe 39 with electrodes 26 in an expanded form, such as by being advanced out of a tubular shaft lumen 80 at a distal end 36 of a tube 31, in accordance with an embodiment of the present invention.

FIG. 2B shows a medical probe 39 with electrodes 26 in a collapsed form within tube 31. In the expanded form (FIG. 2A), the spines 22 bow radially outwardly and in the collapsed form (FIG. 2B) the spines 22 are arranged generally along a longitudinal axis 86 of tubular shaft 84.

As shown in FIG. 2A, the medical probe 39 includes a plurality of flexible spines 22 that are formed at the end of a tubular shaft 84 and are connected at both ends. During a medical procedure, medical professional 34 can deploy medical probe 39 by extending tubular shaft 84 from tube 31 causing the medical probe 39 to exit the tube 31 and transition to the expanded form. Spines 22 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

As will be appreciated by one skilled in the art with the benefit of this disclosure, the basket assembly 28 shown in FIGs. 2A-2B having spines 22 formed from a single sheet of planar material and converging at a central intersection is offered merely for illustrative purposes and the disclosed technology can be applicable to other configurations of basket assemblies 28. For example, the disclosed technology can be applicable to basket assemblies 28 formed from a single spine 22 or multiple spines 22 with each spine 22 being attached at both ends. In other examples, the basket assembly 28 can include a central hub connecting the multiple spines 22 together at a distal end 94 of the basket assembly 28. In yet other examples, the basket assembly 28 can include a single spine 22 configured to form a spiral, multiple spines 22 configured to form a spiral, multiple spines 22 configured to form a tripod or multiple tripods, or any other shape of basket assembly 28. Thus, although FIGs. 2A-2B illustrate a specific configuration of basket assembly 28, the disclosed technology should not be construed as so limited. As well, the basket assembly 28 can be formed by laser cutting a cylindrical hollow stock material whereby the laser is mounted for rotation about the longitudinal axis (and translation thereto) of the cylindrical stock while cutting.

The spines 22 can be formed from a single sheet of planar material to form a generally star shape. In other words, the spines 22 can be formed from the single sheet of planar material such that the spines 22 converge toward a central intersection. The intersection can be a solid piece of material or include one or more apertures.

As will be appreciated, the spine 22 can be electrically isolated from the electrode 26 to prevent arcing from the electrode 26 to the spine 22. For example, insulative jackets can be positioned between the spine 22 and the electrode 26, but one of skill in the art will appreciate that other insulative coverings are contemplated. For example, an insulative coating can be applied to the spine 22, the electrodes 26, or both. The insulative jackets can be made from a biocompatible, electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets may also include one or more additives or fillers, such as, for example, polytetrafluoroethylene (PTFE), boron nitride, silicon nitride, silicon carbide, aluminum oxide, aluminum nitride, zinc oxide, and the like.

In embodiments described herein, electrodes 26 can be configured to deliver ablation energy (RF and/or IRE) to tissue in heart 12. In addition to using electrodes 26 to deliver ablation energy, the electrodes can also be used to determine the location of medical probe 39 and/or to measure a physiological property such as local surface electrical potentials at respective locations on tissue in heart 12. The electrodes 26 can be biased such that a greater portion of the electrode 26 faces outwardly from the medical probe 39 such that the electrodes 26 deliver a greater amount of electrical energy outwardly away from the medical probe 39 (i.e., toward the heart 12 tissue) than inwardly toward the medical probe 39.

Examples of materials ideally suited for forming electrodes 26 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

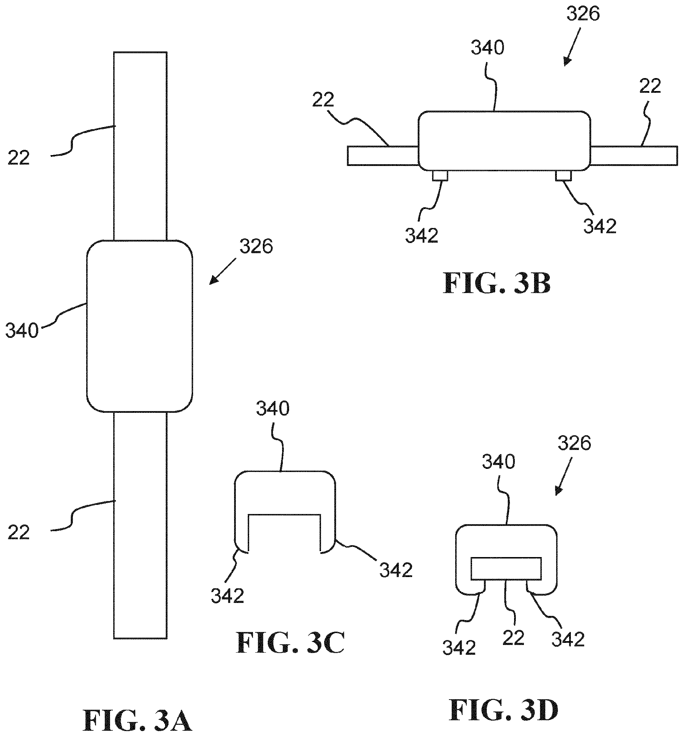

FIG. 3A is a schematic pictorial illustration showing a top view of a spine 22 with an electrode 326 attached thereto, FIG. 3B is a schematic pictorial illustration showing a side view of a spine 22 with an electrode body 340 of the medical probe 39 to illustrate how the electrode 326 can be attached to the spine 22,FIG. 3C is a schematic pictorial illustration showing a front view of an electrode 326 with legs 342, and FIG. 3D is a schematic pictorial illustration showing a front view of an electrode body 340 with legs 342 crimped onto a spine 22 of a medical probe 39, in accordance with the disclosed technology.

As shown in FIGs. 3B-3D, legs 342 can be attached to the electrode body 340. The legs 342 can be configured to be bent or otherwise deformed at least partially around the spine 22 to secure the electrode 326 to the spine 22.

In some examples, the legs 342 can be crimped into the spine 22 to further secure the electrode 326 to the spine 22. For example, the legs 342 can be pointed so as to allow them to dig into the spine 22 for added security. Each of the legs 342 can taper from an end attached to the electrode body 340 to a pointed end opposite the electrode body 340. In other examples, a first leg 342 can overlap with a second leg 342 around the spine 22 to engage the second leg 342. The first leg 342 can be positioned opposite the second leg 342 on the electrode body 340. To illustrate further, the legs 342 can include tongue-and-groove type unions, or any other suitable union type, that do not allow the legs 342 to disengage from one another once engaged.

The electrode 326 can include as few as one and up to twelve legs 342. If the electrode 326 includes only a single leg 342, the single leg 342 can be configured to extend from a first side all the way to a second side of the electrode body 340 when bent around the spine 22. As another example, the number of legs 342 can include four legs 342 and the legs 342 can be dispersed evenly on opposite sides of the electrode body 340. In other examples, the number of legs 342 can be different on either side of the electrode body 340 so as to allow the legs 342 to not overlap when crimped around or into the spine 22. Generally, fewer (for example two or fewer) legs 342 allow for the spine 22 to flex more freely when secured to the spine 22 as compared to a greater number of legs 342 (for example four or more) which allow the spine 22 to flex less.

The electrode body 340 can in some examples be rounded so as to reduce drag and/or snagging on tissue or devices. This can for example, facilitate easier retraction of the medical probe 39 into the tube 31.

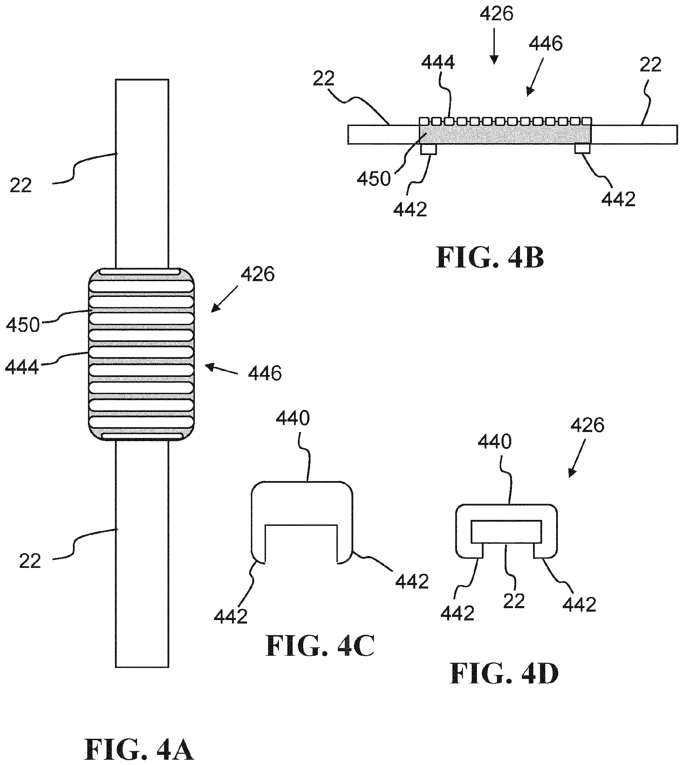

FIGS. 4A-4D illustrate an electrode 426 having an undulating outer surface 446. FIG. 4A is a top view while FIG. 4B is a side view of a spine 22 and an electrode 426 having an electrode body 440 with an undulating outer surface 446. FIGs. 4C and 4D illustrate a front view of the electrode 426 and legs 442 of the electrode 426 being bent around the spine 22. As will be explained further herein, the electrode 426 can further include a flexible material coating 450 and windows 444 formed into the flexible material coating 450.

The undulating outer surface 446 can be configured to permit the electrode body 440 to bend. The electrode 426 can include an electrode body 440 configured to conduct electrical energy for ablation and/or mapping of electrical signals in tissue of a body part. For example, the electrode 426 can be configured to ablation or mapping of cardiac tissue. The undulating outer surface 446 can permit the electrode body 440 to bend by having a plurality of depressions and ridges along an outer surface such that the electrode is better able to bend. In other words, the undulating outer surface 446 can have a sinusoidal or semi-sinusoidal profile when viewed from a side of the electrode 426. Although only a single side (outwardly-facing side when assembled in the basket assembly 28) of the electrode 426 is shown as having an undulating outer surface 446, one of skill in the art will appreciate that two or more sides of the electrode 426 can have an undulating outer surface 446.

The electrode 426 can have a flexible material coating 450 that can serve to prevent blood from clotting in the depressions formed in the undulating outer surface 446 of the electrode body 440. The flexible material coating 450 can be a polymer material or other suitable material that can bend with the electrode 426 when it is bent while also being biocompatible for insertion into a body. The flexible material coating 450 can be added to the electrode body 426 by spraying, dipping, printing, wrapping, or any other suitable manufacturing method depending on the particular application.

To ensure the electrode 426 is capable of conducting electrical energy between tissue in a body, the electrode 426 can include one or more window 444 formed into the flexible material coating 450. The windows 444 can be sized to allow sufficient contact between the electrode 426 and tissue. The windows 444 can be formed by laser cutting, chemically etching, mechanically cutting, or otherwise removing the flexible material coating 450 at selected locations along the electrode 426. For example, the windows 444 can be formed into the flexible material coating 450 at locations between recesses formed in the undulating outer surface 446. In this way, the flexible material coating 450 can fill in the recesses of the undulating outer surface 446 to prevent blood clots in those locations.

Similar to the electrode 326, the electrode 426 can further include one or more legs 442 attached to the electrode body 440 and configured to bend at least partially around a spine 22 of a basket catheter 28 to attach the electrode body 340 to the spine 22. The one or more legs 442 can extend from an edge of the electrode body 340 and can be configured to be bent such that the electrode is crimped to the spine 22. By attaching the legs 442 to the electrode body 440 where the thicker portions of the undulating outer surface 446 are present, the electrode body 340 can still be permitted to bend as previously described. In other words, the legs 442 can be aligned with ridges of the undulating outer surface 446 rather than depressions of the undulating outer surface 446. In other examples, the legs 442 can be aligned with depressions of the undulating outer surface 446 to permit the electrode 426 to have more flexibility.

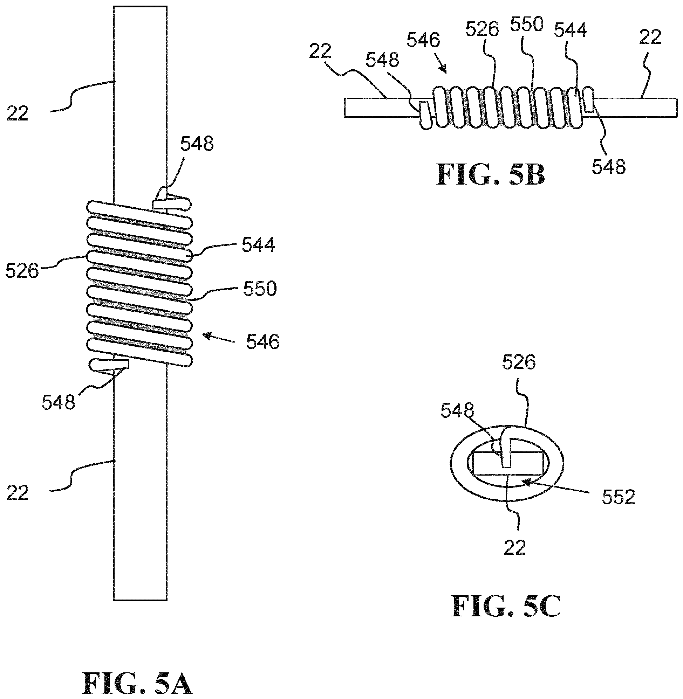

FIGs. 5A-5C illustrate another example of an electrode 526 having an undulating outer surface 546. In particular, FIG. 5A illustrates a top view, FIG. 5B is a side view, and FIG. 5C is a front view of a spine 22 and a spiral wound wire electrode 526., The spiral wound wire electrode 526 can be attached to the spine 22 by being wound around the spine 22 or wound separate from the spine 22 and then slid over the spine 22 when assembled. Similar to the electrode 426, the spiral wound wire electrode 526 can be coated with a flexible material 550 filling in recesses in the spiral wound wire electrode 526. Furthermore, similar to the electrode 426, the spiral wound wire electrode 526 can include one or more windows 544 cut into the flexible material 550 exposing the spiral wound wire electrode 526. The flexible material 550 can in some examples serve to secure the spiral wound wire electrode 526 to the spine 22. Furthermore, the ends 548 of the spiral wound wire electrode 526 can be configured to dig into, or otherwise mechanically engage, the spine 22. For example, the ends 548 of the spiral wound wire electrode 526 can be bent at an angle into the spine 22 to engage the spine 22 and secure the spiral wound wire electrode 526 to the spine 22.

In some examples such as that of FIG. 5C, the spiral wound wire 526 can be wound in an oblong helical pattern with a pitch that allows an area of the spiral wound wire electrode 526 sufficiently large enough to allow the spiral wound wire electrode 526 to map and/or ablate tissue in a heart 12. As shown a gap 552 can exist between the spiral wound wire electrode 526 and the spine 22 through which electrical wires, irrigation tubing, or other items can be disposed. This disclosure is intended to include oblong helical patterns of the spiral wound wire electrode 526 optimized for the application of various types of minimally invasive energy applications as will be appreciated by those skilled in the pertinent art. In other examples, the spiral wound wire electrode 526 can be two electrode wires wound around the spine 22 together. For example, the two electrode wires can be wound around the spine with each other and one electrode can be configured for mapping of tissue while can be configured for ablation of tissue. In still other examples, a first electrode wire can be wound around the spine 22 while a second electrode wire is wound around the outside of the first electrode. The first electrode wire can act as a reference electrode to reduce noise in the electrical signals. As yet another example, the first electrode can be configured to function as a single axis sensor and be used to determine a position and an orientation of the basket catheter 28.

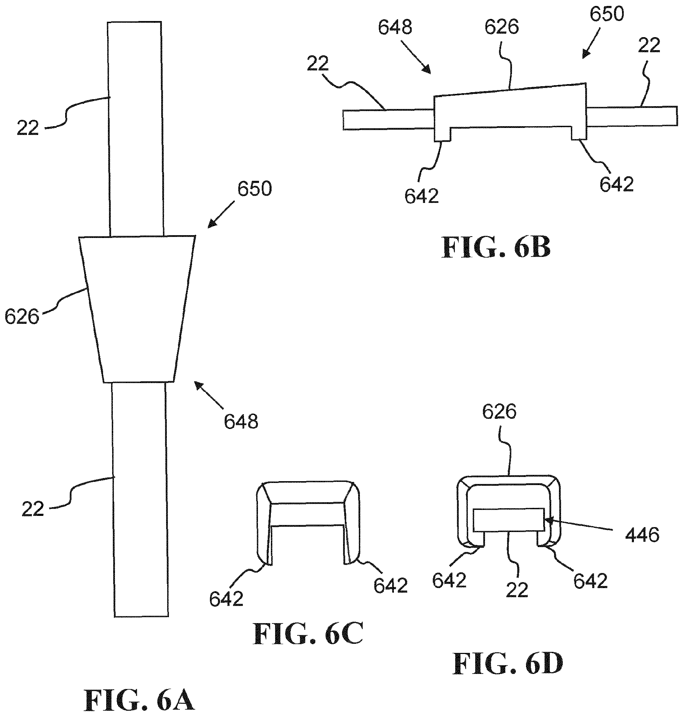

FIG. 6A is a top view while FIG. 6B is a side view of a spine 22 with an electrode 626 tapering outwardly from a proximal end 648 to a distal end 650, in accordance with the disclosed technology. For example, the electrode 626 can include a proximal end 648 having a first thickness and a distal end 650 having a second thickness. The second thickness can be greater than the first thickness. The electrode 626 can taper from the first thickness to the second thickness between the proximal end 648 and the distal end 650. The first thickness and the second thickness can each be measured in a height direction of the electrode 26 and/or in a width direction of the electrode 626 depending on the particular configuration.

In some examples, the electrode 626 can be oriented with the proximal end 648 being smaller than the distal end 650 so as to facilitate easier retraction of the medical probe 39 into the tube 31. The angle at which the electrode 626 tapers from the proximal end 648 to the distal end 650 can be varied to ensure the electrode 626 is able to retract easily into the tube 31 while also having sufficient surface area for mapping and/or ablation of tissue.

FIGs. 6C and 6D illustrate the electrode 626 having legs 642 that can be bent around the spine 22 in accordance with the examples described further herein. The one or more legs 642 can include two or more legs 642 attached to the electrode 26 and be configured to bend at least partially around the spine 22 of a basket catheter 28 so as to be attached thereto. The one or more legs 642 can extend from an edge of the electrode 626 and can be configured to be bent such that the electrode is crimped to the spine 22.

Although not shown, it will be appreciated that the electrode 626 can further include an undulating outer surface 446 as described previously herein. In other words, the various examples of electrodes described herein include features that can be incorporated into an electrode to meet a particular design consideration. For example, the electrode may have legs 642, and/or an undulating outer surface 446, and/or a tapering body (e.g., electrode 626) as desired for a particular application.

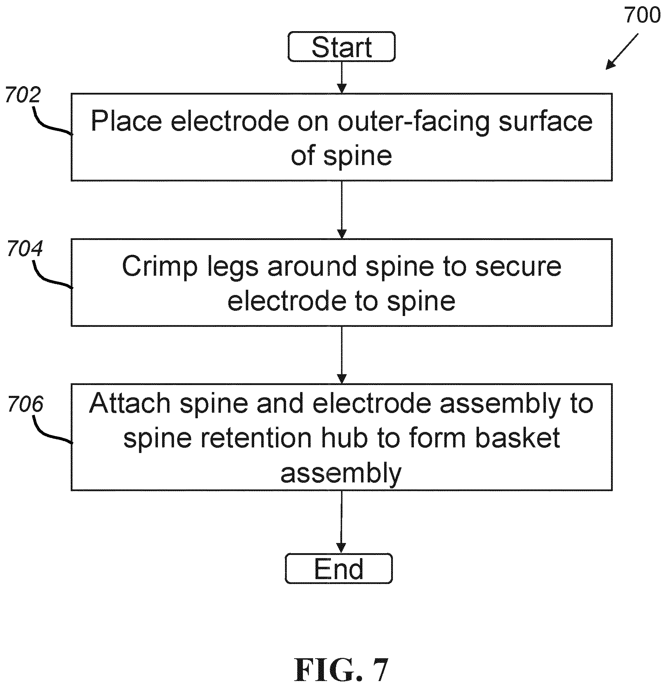

FIG. 7 is a flowchart illustrating a method 700 of attaching an electrode (e.g., electrode 26, 326, 426, 526, and/or 626) to a spine (e.g., spine 22), in accordance with an embodiment of the present invention. The method 700 can include placing 702 an electrode body (e.g., electrode body 340) against a spine with one or more legs (e.g., legs 342) attached to the electrode body extending beyond the spine. The method 700 can include crimping 704 the one or more legs around the spine to secure the electrode to the spine. The one or more legs can extend from an edge of the electrode body and can be configured to be bent such that the electrode is crimped to the spine in accordance with the technology described herein. Furthermore, the electrode can include any of the features of the electrode described here. For example, the electrode body can further include a proximal end having a first thickness and a distal end having a second thickness The second thickness can be greater than the first thickness. The electrode body can taper from the first thickness to the second thickness between the proximal end and the distal end. The first thickness and the second thickness can each be measured in a height direction of the electrode body. Alternatively, the first thickness and the second thickness can each be measured in a width direction of the electrode body. As another example, the electrode body can further include an undulating outer surface. The undulating outer surface can be configured to permit the electrode body to bend. The method 700 can further include attaching 706 the spine and electrode assembly to a spine retention hub to form a basket assembly (e.g. basket assembly 28).

The electrodes described in relation to method 700 can be configured to deliver electrical pulses having a peak voltage of at least 900 volts (V). The spine can include a material selected from a group including of nitinol, cobalt chromium, stainless steel, titanium. Alternatively, or in addition, the spine can include a polymer. The electrode can include a ring type electrode, a bulging type electrode, or a rectangular electrode. The electrodes can be configured to deliver electrical pulses for irreversible electroporation, the pulses having a peak voltage of at least 900 volts (V).

The electrode can be configured to deliver electrical pulses having a peak voltage of at least 900 volts (V). The electrode can be configured to deliver electrical pulses having a peak voltage of at least 900 volts (V).

As will be appreciated, the method 700 just described can be varied in accordance with the various elements and implementations described herein. That is, methods in accordance with the disclosed technology can include all or some of the steps described above and/or can include additional steps not expressly disclosed above. Further, methods in accordance with the disclosed technology can include some, but not all, of a particular step described above. Further still, various methods described herein can be combined in full or in part. That is, methods in accordance with the disclosed technology can include at least some elements or steps of a first method and at least some elements or steps of a second method.

The embodiments described above are cited by way of example, and the present invention is not limited by what has been particularly shown and described hereinabove. Rather, the scope of the invention is defined by the independent claim.

## Claims

1. An electrode (326) for a medical probe, the electrode comprising:
an electrode body (340) configured to deliver electrical energy to biological tissues; and **characterized by**
one or more legs (342) attached to the electrode body and configured to bend at least partially around or into a spine of a basket catheter so as to be attached thereto.

2. The electrode of claim 1, the one or more legs extending from an edge of the electrode body and configured to be bent such that the electrode is crimped to the spine.

3. The electrode of claim 1 or claim 2, the electrode body further comprising a proximal end having a first thickness and a distal end having a second thickness, the second thickness being greater than the first thickness.

4. The electrode of claim 3, the electrode body tapering from the first thickness to the second thickness between the proximal end and the distal end.

5. The electrode of claim 3 or claim 4, wherein the first thickness and the second thickness are each measured in a height direction of the electrode body.

6. The electrode of claim 3 or claim 4, wherein the first thickness and the second thickness are each measured in a width direction of the electrode body.

7. The electrode of any preceding claim, wherein the electrode is configured to deliver electrical pulses having a peak voltage of at least 900 volts (V).

8. The electrode of any preceding claim, the electrode body further comprising an undulating outer surface.

9. The electrode of claim 8, wherein the undulating outer surface is configured to permit the electrode body to bend.

10. The electrode of claim 3 or any of claims 4 - 9 when dependent on claim 3, the one or more legs comprising two or more legs attached to the electrode body and configured to bend at least partially around the spine of a basket catheter so as to be attached thereto.

11. The electrode of claim 10, the one or more legs extending from an edge of the electrode body and configured to be bent such that the electrode is crimped to the spine.

12. A medical probe, comprising:
an insertion tube having a proximal end and a distal end, the insertion tube extending along a longitudinal axis; and
an expandable basket assembly (28) coupled to the distal end of the insertion tube, the expandable basket assembly comprising:
a plurality of spines (22) extending along the longitudinal axis and configured to bow radially outward from the longitudinal axis when the expandable basket assembly is transitioned from a collapsed form to an expanded form; and
a plurality of electrodes according to any preceding claim, wherein each electrode of the plurality of electrodes is attached to a spine of the plurality of spines by bending the one or more legs attached to the electrode body at least partially around the spine of the plurality of spines.

## Patentansprüche

1. Elektrode (326) für eine medizinische Sonde, wobei die Elektrode umfasst:
einen Elektrodenkörper (340), der konfiguriert ist, um elektrische Energie an biologische Gewebe abzugeben; und **gekennzeichnet durch**
ein oder mehrere Beine (342), die an dem Elektrodenkörper befestigt sind und konfiguriert sind, um sich zumindest teilweise um eine Wirbelsäule eines Korbkatheters herum oder in diese hinein zu biegen, um daran befestigt zu werden.

2. Elektrode nach Anspruch 1, wobei sich das eine oder die mehreren Beine von einer Kante des Elektrodenkörpers erstrecken und konfiguriert sind, um gebogen zu werden, so dass die Elektrode an die Wirbelsäule gecrimpt wird.

3. Elektrode nach Anspruch 1 oder Anspruch 2, wobei der Elektrodenkörper ferner ein proximales Ende mit einer ersten Dicke und ein distales Ende mit einer zweiten Dicke umfasst, wobei die zweite Dicke größer als die erste Dicke ist.

4. Elektrode nach Anspruch 3, wobei sich der Elektrodenkörper von der ersten Dicke zu der zweiten Dicke zwischen dem proximalen Ende und dem distalen Ende verjüngt.

5. Elektrode nach Anspruch 3 oder Anspruch 4, wobei die erste Dicke und die zweite Dicke jeweils in einer Höhenrichtung des Elektrodenkörpers gemessen werden.

6. Elektrode nach Anspruch 3 oder Anspruch 4, wobei die erste Dicke und die zweite Dicke jeweils in einer Breitenrichtung des Elektrodenkörpers gemessen werden.

7. Elektrode nach einem der vorstehenden Ansprüche, wobei die Elektrode konfiguriert ist, um elektrische Impulse mit einer Spitzenspannung von mindestens 900 Volt (V) zu liefern.

8. Elektrode nach einem der vorstehenden Ansprüche, wobei der Elektrodenkörper ferner eine wellenförmige Außenoberfläche umfasst.

9. Elektrode nach Anspruch 8, wobei die wellenförmige Außenoberfläche konfiguriert ist, um dem Elektrodenkörper zu ermöglichen, sich zu biegen.

10. Elektrode nach Anspruch 3 oder einem der Ansprüche 4 bis 9, wenn abhängig von Anspruch 3, wobei das eine oder die mehreren Beine zwei oder mehr Beine umfassen, die an dem Elektrodenkörper befestigt und konfiguriert sind, um sich zumindest teilweise um die Wirbelsäule eines Korbkatheters zu biegen, um daran befestigt zu werden.

11. Elektrode nach Anspruch 10, wobei sich das eine oder die mehreren Beine von einer Kante des Elektrodenkörpers erstrecken und konfiguriert sind, um gebogen zu werden, so dass die Elektrode an die Wirbelsäule gecrimpt wird.

12. Medizinische Sonde, umfassend:
ein Einführrohr, das ein proximales Ende und ein distales Ende aufweist, wobei sich das Einführrohr entlang einer Längsachse erstreckt; und
eine ausdehnbare Korbbaugruppe (28), die mit dem distalen Ende des Einführrohrs gekoppelt ist, die ausdehnbare Korbbaugruppe umfassend:
eine Vielzahl von Dornen (22), die sich entlang der Längsachse erstrecken und konfiguriert sind, sich radial nach außen von der Längsachse zu wölben, wenn die expandierbare Korbbaugruppe von einer zusammengefalteten Form in eine expandierte Form überführt wird; und
eine Vielzahl von Elektroden nach einem der vorstehenden Ansprüche, wobei jede Elektrode der Vielzahl von Elektroden an einem Dorn der Vielzahl von Dornen durch Biegen des einen oder der mehreren an den Elektrodenkörper angebrachten Beine zumindest teilweise um den Dorn der Vielzahl von Dornen befestigt ist.

## Revendications

1. Électrode (326) pour une sonde médicale (22), l'électrode comprenant :
un corps d'électrode (340) configuré pour délivrer de l'énergie électrique aux tissus biologiques ; et **caractérisée par**
une ou plusieurs jambes (342) fixées au corps d'électrode et conçues pour se courber au moins partiellement autour ou dans une colonne vertébrale d'un cathéter à panier afin d'y être fixées.

2. Électrode selon la revendication 1, la ou les jambes s'étendant à partir d'un bord du corps d'électrode et conçues pour être courbées de telle sorte que l'électrode est sertie sur la colonne vertébrale.

3. Électrode selon la revendication 1 ou la revendication 2, le corps d'électrode comprenant en outre une extrémité proximale présentant une première épaisseur et une extrémité distale présentant une seconde épaisseur, la seconde épaisseur étant supérieure à la première épaisseur.

4. Électrode selon la revendication 3, le corps d'électrode s'éffilant de la première épaisseur à la seconde épaisseur entre l'extrémité proximale et l'extrémité distale.

5. Électrode selon la revendication 3 ou la revendication 4, dans laquelle la première épaisseur et la seconde épaisseur sont chacune mesurées dans une direction de hauteur du corps d'électrode.

6. Électrode selon la revendication 3 ou la revendication 4, dans laquelle la première épaisseur et la seconde épaisseur sont chacune mesurées dans une direction de largeur du corps d'électrode.

7. Électrode selon l'une quelconque revendication précédente, dans laquelle l'électrode est configurée pour délivrer des impulsions électriques présentant une tension de crête d'au moins 900 volts (V).

8. Électrode selon l'une quelconque revendication précédente, le corps d'électrode comprenant en outre une surface extérieure ondulée.

9. Électrode selon la revendication 8, dans laquelle la surface extérieure ondulée est conçue pour permettre au corps d'électrode de se courber.

10. Électrode selon la revendication 3 ou l'une quelconque des revendications 4 à 9 lorsqu'elle dépend de la revendication 3, la ou les jambes comprenant deux jambes ou plus fixées au corps d'électrode et conçues pour se courber au moins partiellement autour de la colonne vertébrale d'un cathéter à panier afin d'y être fixées.

11. Électrode selon la revendication 10, la ou les jambes s'étendant à partir d'un bord du corps d'électrode et conçues pour être courbées de telle sorte que l'électrode est sertie sur la colonne vertébrale.

12. Sonde médicale, comprenant :
un tube d'insertion présentant une extrémité proximale et une extrémité distale, le tube d'insertion s'étendant le long d'un axe longitudinal ; et
un ensemble panier dilatable (28) accouplé à l'extrémité distale du tube d'insertion, l'ensemble panier dilatable comprenant :
une pluralité de colonnes vertébrales (22) s'étendant le long de l'axe longitudinal et conçues pour s'incliner radialement vers l'extérieur à partir de l'axe longitudinal lorsque l'ensemble panier dilatable passe d'une forme repliée à une forme dilatée ; et
une pluralité d'électrodes selon l'une quelconque revendication précédente, dans laquelle chaque électrode de la pluralité d'électrodes est fixée à une colonne vertébrale de la pluralité de colonnes vertébrales en courbant la ou les jambes fixées au corps d'électrode au moins partiellement autour de la colonne vertébrale de la pluralité de colonnes vertébrales.
